# EUROPEAN PATENT APPLICATION

(11) **EP 1 591 455 A1**
(43) Date of publication of application: **02.11.2005**
(21) Application number: 04291107.3
(22) Date of filing: 29.04.2004
(51) Int. Cl.: C07K 14/705, G01N 33/94

(54) **Essential tremor diagnostic and treatment**

(71) Applicant: INSTITUT NATIONAL DE LA SANTE ET DE LA RECHERCHE MEDICALE (INSERM), 75013 Paris (FR)
(72) Inventor: Sokoloff, Pierre, 95130 Le Plessis-Bouchard (FR); Lucotte, Gérard, 78700 Conflans Sainte-Honorine (FR); Jeanneteau, Freddy, 75013 Paris (FR); Funalot, Benoît, 94300 Vincennes (FR)
(74) Representative: Chajmowicz, Marion

(57) **Abstract**

The invention relates to an *in vitro* method for diagnosing essential tremor wherein a mutation of dopamine D3 receptor gene or protein is sought. The invention further relates to the use of a ligand having anti-dopamine D3 receptor activity for treatment of essential tremor.

SEQUENCE LISTING

<110>  INSERM

<120>  Essential tremor diagnostic and treatment

<130>  BET 04P0194

<160>  4

<170>  PatentIn version 3.1

<210>  1
<211>  1272
<212>  DNA
<213>  Homo sapiens

<400>  1
tgggctatgg catctctgag tcagctgagt agccacctga actacacctg tggggcagag      60

aactccacag gtgccagcca ggcccgccca catgcctact atgccctctc ctactgcgcg     120

ctcatcctgg ccatcgtctt cggcaatggc ctggtgtgca tggctgtgct gaaggagcgg     180

gccctgcaga ctaccaccaa ctacttagta gtgagcctgg ctgtggcaga cttgctggtg     240

gccaccttgg tgatgccctg ggtggtatac ctggaggtga caggtggagt ctggaatttc     300

agccgcattt gctgtgatgt ttttgtcacc ctggatgtca tgatgtgtac agccagcatc     360

cttaatctct gtgccatcag catagacagg tacactgcag tggtcatgcc cgttcactac     420

cagcatggca cgggacagag ctcctgtcgg cgcgtggccc tcatgatcac ggccgtctgg     480

gtactggcct ttgctgtgtc ctgccctctt ctgtttggct ttaataccac aggggacccc     540

actgtctgct ccatctccaa ccctgatttt gtcatctact cttcagtggt gtccttctac     600

ctgccctttg gagtgactgt ccttgtctat gccagaatct atgtggtgct gaaacaaagg     660

agacggaaaa ggatcctcac tcgacagaac agtcagtgca acagtgtcag gcctggcttc     720

ccccaacaaa ccctctctcc tgacccggca catctggagc tgaagcgtta ctacagcatc     780

tgccaggaca ctgccttggg tggaccaggc ttccaagaaa gaggaggaga gttgaaaaga     840

gaggagaaga ctcggaattc cctgagtccc accatagcgc ctaagctcag cttagaagtt     900

cgaaagctca gcaatggcag attatcgaca tctttgaagc tggggcccct gcaacctcgg     960

ggagtgccac ttcgggagaa gaaggcaacc caaatggtgg ccattgtgct tggggccttc    1020

attgtctgct ggctgccctt cttcttgacc catgttctca atacccactg ccagacatgc    1080

cacgtgtccc cagagcttta cagtgccacg acatggctgg gctacgtgaa tagcgccctc    1140

aaccctgtga tctataccac cttcaatatc gagttccgga aagccttcct caagatcctg    1200

tcttgctgag ggagcagaag agggaacact ctttgtaccc atttctagct gccaggctgt    1260

tggcccactc ag                                                        1272

<210> 2
<211> 398
<212> PRT
<213> Homo sapiens

<400> 2

Met Ala Ser Leu Ser Gln Leu Ser Ser His Leu Asn Tyr Thr Cys Gly
1               5                   10                  15

Ala Glu Asn Ser Thr Gly Ala Ser Gln Ala Arg Pro His Ala Tyr Tyr
            20                  25                  30

Ala Leu Ser Tyr Cys Ala Leu Ile Leu Ala Ile Val Phe Gly Asn Gly
        35                  40                  45

Leu Val Cys Met Ala Val Leu Lys Glu Arg Ala Leu Gln Thr Thr Thr
    50                  55                  60

Asn Tyr Leu Val Val Ser Leu Ala Val Ala Asp Leu Leu Val Ala Thr
65                  70                  75                  80

Leu Val Met Pro Trp Val Val Tyr Leu Glu Val Thr Gly Gly Val Trp
                85                  90                  95

Asn Phe Ser Arg Ile Cys Cys Asp Val Phe Val Thr Leu Asp Val Met
            100                 105                 110

Met Cys Thr Ala Ser Ile Leu Asn Leu Cys Ala Ile Ser Ile Asp Arg
        115                 120                 125

Tyr Thr Ala Val Val Met Pro Val His Tyr Gln His Gly Thr Gly Gln
    130                 135                 140

Ser Ser Cys Arg Arg Val Ala Leu Met Ile Thr Ala Val Trp Val Leu
145                 150                 155                 160

Ala Phe Ala Val Ser Cys Pro Leu Leu Phe Gly Phe Asn Thr Thr Gly
                165                 170                 175

Asp Pro Thr Val Cys Ser Ile Ser Asn Pro Asp Phe Val Ile Tyr Ser
            180                 185                 190

Ser Val Val Ser Phe Tyr Leu Pro Phe Gly Val Thr Val Leu Val Tyr
        195                 200                 205

Ala Arg Ile Tyr Val Val Leu Lys Gln Arg Arg Arg Lys Arg Ile Leu
    210                 215                 220

Thr Arg Gln Asn Ser Gln Cys Asn Ser Val Arg Pro Gly Phe Pro Gln
225                 230                 235                 240

Gln Thr Leu Ser Pro Asp Pro Ala His Leu Glu Leu Lys Arg Tyr Tyr
                245                 250                 255

Ser Ile Cys Gln Asp Thr Ala Leu Gly Gly Pro Gly Phe Gln Glu Arg
            260                 265                 270

Gly Gly Glu Leu Lys Arg Glu Glu Lys Thr Arg Asn Ser Leu Ser Pro
        275                 280                 285

Thr Ile Ala Pro Lys Leu Ser Leu Glu Val Arg Lys Leu Ser Asn Gly
    290                 295                 300

Arg Leu Ser Thr Ser Leu Lys Leu Gly Pro Leu Gln Pro Arg Gly Val
305                 310                 315                 320

Pro Leu Arg Glu Lys Lys Ala Thr Gln Met Val Ala Ile Val Leu Gly
                325                 330                 335

Ala Phe Ile Val Cys Trp Leu Pro Phe Phe Leu Thr His Val Leu Asn
            340                 345                 350

Thr His Cys Gln Thr Cys His Val Ser Pro Glu Lys Ala Thr Thr Trp
        355                 360                 365

Leu Gly Tyr Val Asn Ser Ala Leu Asn Pro Val Ile Tyr Thr Thr Phe
    370                 375                 380

Asn Ile Glu Phe Arg Lys Ala Phe Leu Lys Ile Leu Ser Cys
385                 390                 395

<210>  3
<211>  20
<212>  DNA
<213>  Artificial Sequence

<220>
<223>  primer

<400>  3
gctctatctc aactctcaca                                                  20

<210>  4

<211>  21
<212>  DNA
<213>  Artificial Sequence

<220>
<223>  primer

<400>  4
aagtctactc acctccaggt a                                                21

## Description

The invention relates to a method for diagnosing essential tremor and to the treatment of the disease.

Essential tremor (ET) is the most prevalent adult movement disorder (Louis et al., 1996). The prevalence estimates in different studies worldwide range from 0.4 % to 3.9 % for the general population and the prevalence increases with age affecting at least 5 % for the age group above 60 years (Louis et al., 1998).

Essential tremor is a progressive disorder exhibiting variable expression, the severity of tremor varying considerably from mild form to a high amplitude tremor. ET is most frequently characterized by an action (kinetic and postural) tremor of the arms, hands and voice, and less frequently by tremor of the head. The legs are rarely affected. ET can become quite debilitating by causing severe disfiguring head and postural tremor, as well as disabling fine motor hand control and can thus lead to psychosocial problems. Essential tremor is worsened by stimulating factors such as caffeine, anxiety or stress. Current treatments of ET are only partially effective, β-blockers (such as propanolol) and primidone having limited effectiveness.

Essential tremor is a syndrome for which multiple etiologies may be identified. Most of these causes have probably hereditary in origin as ET is associated with a positive family history in at least 50 to 70 % of the patients. Transmission is considered to be autosomal dominant, with variable expressivity and high penetrance (Brin et al., 1998). Previous studies of autosomal dominant pedigrees of ET have identified two disease loci: in a first study of 75 affected individuals from 16 Icelandic families (Gulcher et al., 1997), a gene for familial ET (ETM1) was mapped to chromosome 3q 13 with a maximized overall LOD score > 3.7; the LOD scores, obtained from independent analysis of the families, ranged from -0.720 to 1.290 (with a penetrance of 0.90) with 10 of 16 families contributing to positive LOD scores. A second locus on chromosome 2p22-25 was subsequently identified (ETM2) by linkage analysis of a large family of Czech descent (Higgins et al., 1997) and later evidence (Higgins et al., 1998) was presented for linkage of three unrelated American families with ET to this second locus. More recently, a chromosome 4p haplotype was reported for Lewy body parkinsonism that segregated among individuals in a pedigree who exhibited only postural tremor consistent with ET (Farrer et al., 1999).

However, up to now, no genetic test is available for a simple diagnostic of essential tremor, for instance by analysing a blood or urine sample. Up to now, the disease is only diagnosed by specialists on the basis of a clinical and neurological examination, which nevertheless makes it possible for an accurate diagnostic of essential tremor.

Anti-parkinsonian agents such as L-DOPA, ropinirol, pramipexol, bromocriptine, are moderately active against essential tremor. Actually, the lack of efficiency of these drugs for reducing tremor is a diagnostic criteria of essential tremor.

Treatments are currently available. However administration thereof is restricted due to their side effects. Alcohol is one of the oldest forms of self-treatment. Other sedatives, such as benzodiazepine and barbituric, may be used but the risks of drug abuse are high and the sedative effects sometimes excessive. Agents with peripheral activity such as β-blockers, especially propanolol, may be administered and represent the better tolerated treatment. However, responses to β-blockers are variable as only a partial symptomatic improvement is observed, furthermore only in about 50 % of the patients. Additionally β-blockers lack efficiency in the most severe forms of ET. The side-effects observed in numerous patients include impotence and depression. Increased cardiovascular risks are also associated with β-blocker treatments.

For the most severe forms of the disease, which are refractory to current medication, deep brain electric stimulation may be used to partly destroy the thalamus. However this invasive surgery may induce many complications.

Therefore both a diagnostic test and therapeutical solutions are acutely required for essential tremor.

The inventors have now identified that mutations of the dopamine D₃ receptor are associated with essential tremor.

Using a candidate gene approach, the inventors have tested the dopamine D₃ receptor gene (DRD3), located in 3q13.3, as a potential mutated ET gene. In particular, they have demonstrated the involvement of allele Gly at the Ser-9-Gly polymorphism of DRD3 in the development of the disease. This finding has important implications as regards to ET diagnosis in patients, and ET treatment with DRD3 antagonists or partial agonists.

In view of their results, the inventors propose an *in vitro* method for diagnosing essential tremor based on the detection of mutations in DRD3 gene or protein, as well as the use of D₃ receptor ligands for treating patients with essential tremor.

### Definitions

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. A "promoter" or "promoter sequence" is a DNA regulatory region capable of binding RNA polymerase in a cell and initiating transcription of a downstream (3' direction) coding sequence. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

As used herein, the term "oligonucleotide" refers to a nucleic acid, generally of at least 10, preferably at least 12, more preferably at least 15, and still preferably at least 20 nucleotides, preferably no more than 100 nucleotides, still preferably no more than 70 nucleotides, and which is hybridizable to a DRD3 genomic DNA, cDNA, or mRNA.

As used herein, an amplification primer is an oligonucleotide for amplification of a target sequence by extension of the oligonucleotide after hybridization to the target sequence or by ligation of multiple oligonucleotides which are adjacent when hybridized to the target sequence. At least a portion of the amplification primer hybridizes to the target. This portion is referred to as the target binding sequence and it determines the target-specificity of the primer. The terms "primer" and "probe" refer to the function of the oligonucleotide. Typically, contrary to a primer, a probe is not extended by polymerase or ligation following hybridization to the target. A hybridized oligonucleotide may thus function as a probe if it is used to capture or detect a target sequence, and the same oligonucleotide may function as a primer when it is employed as a target binding sequence in an amplification primer.

As used herein, the term "dopamine D₃ receptor", "D₃ receptor", or "DRD3" denotes a subtype of dopamine receptor principally expressed in the limbic system (Sokoloff et al., 1990). D₃ receptor has been further described in the international patent application WO 91/15513. In the context of the invention DRD3 may originate form any mammalian species, including rodent, such as rat or mouse, feline, canine, primate, especially monkey or human. Preferably DRD3 is of human origin. Human DRD3 gene was cloned by Giros et al. (1990). The sequence of this gene is deposited in the database Genbank under accession number A19667; it is further described in the sequence shown as SEQ ID No.1 in the sequence listing. The corresponding polypeptide sequence is deposited in databases under accession number CAA01483 (SEQ ID No.2).

The terms "mutant" and "mutation" mean any detectable change in genetic material, e.g. DNA, RNA, cDNA, or any process, mechanism, or result of such a change. This includes gene mutations, in which the structure (e.g. DNA sequence) of a gene is altered, any gene or DNA arising from any mutation process, and any expression product (e.g. protein or enzyme) expressed by a modified gene or DNA sequence. Mutations include deletion, insertion or substitution of one or more nucleotides. The mutation may occur in the coding region of a gene (i.e. in exons), in introns, or in the regulatory regions (e.g. enhancers, response elements, suppressors, signal sequences, polyadenylation sequences, promoters) of the gene. Generally a mutation is identified in a subject by comparing the sequence of a nucleic acid or polypeptide expressed by said subject with the corresponding nucleic acid or polypeptide expressed in a control population. Where the mutation is within the gene coding sequence, the mutation may be a "missense" mutation, where it replaces one amino acid with another in the gene product, or a "non sense" mutation, where it replaces an amino acid codon with a stop codon. A mutation may also occur in a splicing site where it creates or destroys signals for exon-intron splicing and thereby lead to a gene product of altered structure. A mutation in the genetic material may also be "silent", i.e. the mutation does not result in an alteration of the amino acid sequence of the expression product.

Some mutations of human DRD3 gene have been described in the international patent application WO 92/07937, in particular the substitution 31A>G, according to SEQ ID N°1. The mutation 31A>G results in a substitution S9G within the sequence of DRD3 protein shown in SEQ ID N°2. By direct sequencing of human DRD3 gene, the inventors have further identified the mutations 57A>G, 726A>G and 993A>G, according to SEQ ID N°1, which all are synonymous (silent) mutations that do not change the aminoacid sequence in the DRD3 protein. A substitution 118G>A, according to SEQ ID N°1, which results in a A38T substitution in the DRD3 protein, according to SED ID N°2, has been also described (Ishiguri et al., 2000).

In the context of the instant application, mutations identified in DRD3 gene or protein are designated pursuant to the nomenclature of Dunnen and Antonarakis (2000). As defined by Dunnen and Antonarakis at the nucleic acid level, substitutions are designated by ">", e.g. "31A>G" denotes that at nucleotide 31 of the reference sequence a A is changed to a G. Deletions are designated by "del" after the deleted interval (followed by the deleted nucleotides). For instance 1997-1999deI (alternatively 1997-1999-deITTC) denotes a TTC deletion from nucleotides 1997 to 1999. A TG deletion in the sequence ACTGTGTGCC (A is nucleotide 1991) is designated as 1997-1998del (or 1997-1998deITG). Insertions are designated by "ins," followed by the inserted nucleotides. For example, 1997-1998insT denotes that a T was inserted between nucleotides 1997 and 1998. A TG insertion in the TG-tandem repeat sequence of ACTGTGTGCC (A is nucleotide 1991) is described as 1998-1999insTG (where 1998 is the last G of the TG-repeat). Intron mutations are designated by the intron number (preceded by "IVS") or cDNA position; positive numbers starting from the G of the GT splice donor site, whereas negative numbers starting from the G of the AG splice acceptor site. For instance IVS4-2A>C (1998-2A>C) denotes the A to C substitution at nt -2 of intron 4, at the cDNA level positioned between nucleotides 1997 and 1998. IVS4+1 G>T (1997+1 G>T) denotes the G to T substitution at nucleotide+1 of intron 4. When the full-length genomic sequence is known, the mutation is best designated by the nucleotide number of the genomic references. As used herein, allele 1 denotes the DRD3 gene variant with an A at nucleotide 31, according to SEQ ID N° 1. Allele 2 denotes the DRD3 gene variant with a G at nucleotide 31, according to SEQ ID N° 1.

Variations at the protein level describe the consequence of the mutation and are reported as follows. Stop codons are designated by X (e.g. R97X denotes a change of Arg96 to a termination codon). Amino acid substitutions a designated for instant by "S9G", which means that Ser in position 9 is replaced by Gly. Deletions are indicated by "del" after the amino acid interval (e.g. T97-C102 del denotes that amino acids Thr97 to Cys102 are deleted). Insertions are designated by "ins" after the amino acid interval, followed by the inserted amino acids or the number of amino acids inserted (e.g. T97-W98insLQS or T97-W98ins3 denotes that three amino acids are inserted between amino acids 97 and 98). As used herein, the DRD3 variant with a Ser at residue 9, according to SEQ ID N°2 is denoted the ⁹Ser variant. The variant with a Gly at residue 9, according to SEQ ID N°2, is denoted the ⁹Gly variant.

As used herein, the term "subject" denotes a mammal, such as a rodent, a feline, a canine, and a primate. Preferably a subject according to the invention is a human.

### Diagnostic methods

The inventors have demonstrated a genetic link between mutations in the gene encoding the dopamine D3 receptor and essential tremor.

The invention thus relates to an in vitro method for diagnosing essential tremor, or a risk of developing essential tremor, which comprises the step consisting of detecting a mutation in dopamine D3 receptor (DRD3) gene, said mutation being indicative of essential tremor, or of an increased risk of developing essential tremor.

According to a first embodiment a mutation in the DRD3 gene may be sought in a nucleic acid sample from a subject likely to be affected with, or likely to develop, essential tremor.

In particular, the mutation of the DRD3 gene may be a missense mutation, a non sense mutation, a deletion, an insertion, or a combination thereof.

The mutation may be in the coding regions, in introns, or in the regulatory regions of DRD3 gene. Where the mutation is in the coding sequence, said mutation may be sought in exons of DRD3 genomic sequence, or in DRD3 mRNA or cDNA.

Advantageously, the mutation in the DRD3 coding sequence may be selected from the group consisting of substitutions 31A>G, 57A>G, 118G>A, 726A>G and 993A>G, according to SEQ ID N°1. Preferably said mutation in DRD3 gene is a 31A>G substitution in the DRD3 coding sequence as shown in SEQ ID No.1.

Said mutation in DRD3 coding sequence may result in a mutation in the DRD3 protein. Preferably said mutation is selected from the group consisting of S9G and A38T mutations in DRD3 protein, as shown in the sequence SEQ ID No.2. Still preferably said mutation in DRD3 protein is a S9G substitution.

The nucleic acid sample may be obtained from any cell source or tissue biopsy. Non-limiting examples of cell sources available include without limitation blood cells, buccal cells, epithelial cells, fibroblasts, or any cells present in a tissue obtained by biopsy. Cells may also be obtained from body fluids, such as blood, plasma, serum, lymph, etc. DNA may be extracted using any methods known in the art, such as described in Sambrook et al., 1989. RNA may also be isolated, for instance from tissue biopsy, using standard methods well known to the one skilled in the art such as guanidium thiocyanate-phenol-chloroform extraction (Chomocyznski et al., 1987).

DRD3 mutation may be detected in a RNA or DNA sample, preferably after amplification. For instance, the isolated RNA may be subjected to coupled reverse transcription and amplification, such as reverse transcription and amplification by polymerase chain reaction (RT-PCR), using specific oligonucleotide primers that are specific for a mutated site or that enable amplification of a region containing the mutated site. According to a first alternative, conditions for primer annealing may be chosen to ensure specific reverse transcription (where appropriate) and amplification; so that the appearance of an amplification product be a diagnostic of the presence of a particular DRD3 mutation. Otherwise, RNA may be reverse-transcribed and amplified, or DNA may be amplified, after which a mutated site may be detected in the amplified sequence by hybridization with a suitable probe or by direct sequencing, or any other appropriate method known in the art. For instance, a cDNA obtained from RNA may be cloned and sequenced to identify a mutation in DRD3 sequence.

Currently numerous strategies for genotype analysis are available (Antonarakis et al., 1989 ; Cooper et al., 1991 ; Grompe, 1993). Briefly, the nucleic acid molecule may be tested for the presence or absence of a restriction site. When a base substitution mutation creates or abolishes the recognition site of a restriction enzyme, this allows a simple direct PCR test for the mutation. Further strategies include, but are not limited to, direct sequencing, restriction fragment length polymorphism (RFLP) analysis; hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, HOT cleavage; denaturing gradient gel electrophoresis (DGGE), temperature denaturing gradient gel electrophoresis (TGGE), single-stranded conformational polymorphism (SSCP) and denaturing high performance liquid chromatography (Kuklin et al., 1997). Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology (see e.g. Little et al., 1996); and real-time quantitative PCR. Preferably, DNA from a subject is first subjected to amplification by polymerase chain reaction (PCR) using specific amplification primers. However several other methods are available, allowing DNA to be studied independently of PCR, such as the rolling circle amplification (RCA), the Invader™assay, or oligonucleotide ligation assay (OLA). OLA may be used for revealing base substitution mutations. According to this method, two oligonucleotides are constructed that hybridize to adjacent sequences in the target nucleic acid, with the join sited at the position of the mutation. DNA ligase will covalently join the two oligonucleotides only if they are perfectly hybridized (Nickerson et al., 1990).

Oligonucleotides can be labelled according to any technique known in the art, such as with radiolabels, fluorescent labels, enzymatic labels, sequence tags, etc. A labelled oligonucleotide may be used as a probe to detect the presence of a mutated DRD3 nucleic acid. Alternatively, oligonucleotides (one or both of which may be labelled) can be used for amplifying a DRD3 nucleic acid, for instance by PCR (Saiki et al., Science 1988, 239:487), to detect the presence of a mutation. Generally, oligonucleotides are prepared synthetically, preferably on a nucleic acid synthesizer. Accordingly, oligonucleotides can be prepared with non-naturally occurring phosphoester analog bonds, such as thioester bonds, etc.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989).

The conditions of temperature and ionic strength determine the "stringency" of the hybridization. For preliminary screening for homologous nucleic acids, low stringency hybridization conditions, corresponding to a Tm (melting temperature) of 55°C, can be used, e.g., 5x SSC, 0.1 % SDS, 0.25 % milk, and no formamide ; or 30 % formamide, 5x SSC, 0.5 % SDS). Moderate stringency hybridization conditions correspond to a higher Tm, e.g., 40 % formamide, with 5x or 6x SCC. High stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 II.7-11.8). A minimum length for a hybridizable nucleic acid is at least about 10 nucleotides, preferably at least about 15 nucleotides, and more preferably the length is at least about 20 nucleotides.

In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, *"high stringency"* refers to hybridization and/or washing conditions at 68°C in 0.2 X SSC, at 42°C in 50 % formamide, 4 X SSC, or under conditions that afford levels of hybridization equivalent to those observed under either of these two conditions.

The 31A>G substitution creates a restriction site for the enzyme *Msc* I or its isoschizomer Bal I. Therefore, preferably detection of 31A>G substitution may be carried out by PCR amplification and restriction enzyme digestion using Msc I or Bal I, for instance according to the method described by Lannfelt et al. (1992).

Alternatively, said mutation of DRD3 gene may also be sought in a biological sample from a subject likely to be affected with, or likely to develop, essential tremor by detecting the resulting alteration in DRD3 protein.

The mutation is preferably an amino acid substitution. Preferably said mutation in DRD3 protein is selected from the group consisting of S9G, A79V, V189I, and I397T. Preferably said mutation is a S9G substitution in DRD3 protein as shown in the sequence SEQ ID No.2.

Said mutation may be detected according to any appropriate method known in the art. In particular a sample, such as a tissue biopsy, obtained from a subject may be contacted with antibodies specific of a mutated form of DRD3 protein, i.e. antibodies that are capable of distinguishing between a mutated form of DRD3 and the wild-type protein, to determine the presence or absence of a DRD3 specified by the antibody.

Antibodies that specifically recognize a mutated DRD3 protein also make part of the invention. The antibodies are specific of mutated DRD3 protein, that is to say they do not cross-react with the wild-type DRD3 protein.

The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')₂ and F(v). They can also be immunoconjugated, e.g. with a toxin, or labelled antibodies.

Whereas polyclonal antibodies may be used, monoclonal antibodies are preferred for they are more reproducible in the long run.

Procedures for raising "polyclonal antibodies" are also well known. Polyclonal antibodies can be obtained from serum of an animal immunized against mutated DRD3 protein, which may be produced by genetic engineering for example according to standard methods well-known by one skilled in the art. Typically, such antibodies can be raised by administering mutated DRD3 protein subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by Harlow et al. (1988), which is hereby incorporated in the references.

A "monoclonal antibody" in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al., 1988). Monoclonal antibodies (mAbs) may be prepared by immunizing purified mutated DRD3 protein into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also contemplated such as for example using phage display technology to examine naive libraries (from non-immunised animals); see Barbas et al. (1992), and Waterhouse et al. (1993).

Antibodies raised against mutated DRD3 protein may be cross reactive with wild-type DRD3 protein. Accordingly a selection of antibodies specific for mutated DRD3 protein is required. This may be achieved by depleting the pool of antibodies from those that are reactive with the wild-type DRD3 protein, for instance by submitting the raised antibodies to an affinity chromatography against wild-type DRD3 protein.

Alternatively, binding agents other than antibodies may be used for the purpose of the invention. These may be for instance aptamers, which are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

According to another embodiment the invention relates to an in vitro method for diagnosing essential tremor, or a risk of developing essential tremor, which comprises detecting an increase in wild-type or mutated DRD3 protein activity on a test subject compared with a control subject, i.e. a healthy subject or a standard sample, wherein an increased DRD3 protein activity is indicative of essential tremor, or a risk of developing essential tremor.

An "increased activity" of DRD3 in a test subject or a biological sample refers to a higher total DRD3 activity in the test subject or biological sample in comparison with a control. Preferably, although not necessarily, the activity is at least 10%, more preferably at least 20%, even more preferably at least 50%, and still more preferably at least 100% higher in the test subject or sample than in the control.

An increase in DRD3 protein activity may be due to an increase in the level of DRD3 protein expression. A higher expression level of wild-type or mutated DRD3 protein may result for instance from a mutation in a non-coding region of DRD3 gene such as a promoter region of DRD3 gene, or from a mutation in a coding or non-coding gene involved in DRD3 transcription or translation. An increase in DRD3 protein activity may be due also to an increase in the basal activity of DRD3 protein. As used herein the term "basal" activity denotes the activity of wild-type DRD3 protein in a healthy subject, as may be observed when the receptor is activated by binding of an agonist. An increased basal activity may be related for instance to an increased affinity of a ligand for the mutated DRD3 protein as compared with the wild-type DRD3 or to prolonged stimulation of a downstream component of a DRD3-associated pathway, for instance inhibition of cyclic AMP formation (Griffon et al., 1997). An increase in the basal activity of DRD3 protein may be related to a missense mutation in DRD3 gene that results in a gain-of-function mutation in DRD3 protein.

Accordingly the method of the invention may be carried out by assessing the level of expression or activity of DRD3 protein in a test subject and comparing it to the level of expression or activity in a control subject, wherein an increased expression or basal activity of DRD3 protein in the test subject compared with the control subject is indicative of essential tremor, or a risk of developing essential tremor.

The level of expression of DRD3 may be assessed by determining the amount of mRNA that encodes the DRD3 protein in a biological sample, or by determining the concentration of DRD3 protein in a biological sample. The level of activity of DRD3 protein may be assessed by determining the binding affinity of reported DRD3 ligands, for instance.

The gain-of-function of DRD3 may be assessed after administration of a DRD3-selective agonist or partial agonist and measurement of the effect of DRD3 stimulation by functional neuroimaging, for instance using regional blood flow measurement by Positron Emission Tomography (Black et al., 2002). Examples of DRD3 partial agonists include BP 897 (1-(4-(2-Naphthoylamino)butyl)-4-(2-methoxyphenyl)-1A-piperazine, Pilla et al., 1999) and aripiprazole (7-[4-[4-(2, 3 dichlorophenyl)-1-piperazinyl]butoxy]-3, 4-dihydro-2(1H)-quinoline (Lawler et al., 1999). Alternatively, the gain-of-function of DRD3 may be measured by the attenuation of the effect of a DRD3-selective antagonist, also measured by functional neuroimaging. Exemples of DRD3 antagonists include nafadotride (N[(n-butyl-2-pyrrolidinyl)methyl]-1-methoxy-4-cyano naphtalene-2-carboxamide, Sautel et al. 1995), ST 198 ((E)-N-(4-[1,2,3,4-tetrahydroisoquinolin-2-yl]-butyl)-3-phenylacrylamide), SB-277701A (*trans*-*N*-[4-[2-(6-cyano-1,2,3,4-tetrahydroisoquinolin-2-yl)ethyl]cyclohexyl]-4-quinolinecarboxamide, Reavill et al., 2000), S33084 ((3*aR*,9*bS*)-*N*-[4-(8-cyano-1,3*a*,4,9*b*-tetrahydro-3H-benzopyrano[3,4-*c*]pyrrole-2-yl)butyl]'-phenyl) benzamides, Millan et al., 2000).

### Oligonucleotides and diagnostic kits

According to an aspect of the invention, a DRD3 mutation is detected by contacting a nucleic acid sample from the subject with a nucleic acid probe, which is optionally labeled.

Primers may also be useful to amplify or sequence the portion of the DRD3 gene that contains the mutations of interest.

Such probes or primers are "sequence-specific oligonucleotides", i.e. oligonucleotides or nucleic acids that are capable of specifically hybridizing with a portion of the DRD3 gene sequence containing the mutations of interest under conditions of high stringency (Sambrook et al, 1989).

The invention thus relates to the use of an oligonucleotide that specifically hybridizes to or adjacent to a site of mutation of DRD3 gene for the in vitro diagnosis of essential tremor, or of a risk of developing essential tremor.

The invention also provides a kit for diagnosing essential tremor, or a risk of developing essential tremor, comprising an oligonucleotide that specifically hybridizes to or adjacent to a site of mutation of DRD3 gene.

The kits may include the following components :
(i) a probe as above defined, usually made of DNA, and that may be pre-labelled. Alternatively, the probe may be unlabelled and the ingredients for labelling may be included in the kit in separate containers ; and
(ii) hybridization reagents : the kit may also contain other suitably packaged reagents and materials needed for the particular hybridization protocol, including solid-phase matrices, if applicable, and standards.

In another embodiment, the kits may include :
(i) sequence determination or amplification primers : sequencing primers may be pre-labelled or may contain an affinity purification or attachment moiety ; and
(ii) sequence determination or amplification reagents : the kit may also contain other suitably packaged reagents and materials needed for the particular sequencing amplification protocol. In one preferred embodiment, the kit comprises a panel of sequencing or amplification primers, whose sequences correspond to sequences adjacent to at least one of the polymorphic positions, as well as a means for detecting the presence of each polymorphic sequence.

The site of mutation may be in the coding region, in introns, or in the promoter of DRD3 gene. Preferably, the mutation site is selected from the group consisting of nucleotide positions 31, 57, 118, 726, and 993 of SEQ ID No.1. Still preferably the mutation site is nucleotide 31 of SEQ ID No.1. Advantageously, the oligonucleotide according to the invention may be selected from an oligonucleotide comprising, or consisting of, SEQ ID No.3 and SEQ ID No.4.

In a particular embodiment, it is provided a kit which comprises a pair of oligonucleotide primers specific for amplifying all or part of the DRD3 gene comprising at least one of the mutated positions that are identified above. Preferably said kit may comprise a primer pair consisting of oligonucleotides SEQ ID No.3 and SEQ ID No.4. Additionally the invention relates to the use of a pair of oligonucleotides consisting of SEQ ID No.3 and SEQ ID No.4 for amplifying a region of DRD3 gene spanning position 31 of SEQ ID No.1.

According to another embodiment the kit of the invention may comprise an antibody that specifically recognizes a mutation of DRD3 protein.

The mutation is preferably an amino acid substitution. Preferably said mutation in DRD3 protein is selected from the group consisting of S9G and A38T substitutions in the sequence SEQ ID No 2. Preferably said mutation in DRD3 protein is a S9G substitution in the sequence SEQ ID No.2.

### Essential tremor treatment

The invention further provides a method of treating essential tremor, which aims at counteracting the gain-of-function produced by a DRD3 mutation. The method comprises administering a patient in need thereof with a dopamine D3 receptor ligand having anti-D3 receptor activity.

The invention also relates to the use of a dopamine D3 receptor ligand having anti-D3 receptor activity for the manufacture of a medicament intended for the treatment of essential tremor in a patient in need thereof.

Preferably said DRD3 ligand is a DRD3 partial agonist or a DRD3 antagonist.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the term "patient" or "patient in need thereof', is intended for a human or non-human mammal affected or likely to be affected with essential tremor.

The term "D₃ receptor ligand", as used herein, refers to a molecule that has the capacity to bind the dopamine D₃ receptor. Upon binding, a ligand may or may not modify or interfere with the activity of the DRD3. DRD3 ligands include D₃ receptor partial agonists and antagonists.

The term "D₃ receptor partial agonist" is intended for a compound that forms a complex with DRD3 and acts as a mixed agonist-antagonist. *In vitro,* a partial agonist of DRD3 produces in a cell expressing DRD3 an active response, of which the maximal intensity is lower than that produced by dopamine or its full agonist, for instance quinpirole *(trans-(-)-*4aR-4,4a*,* 5,6,7,8,8a,9-octahydro-5-propyl-1 H(or 2H)-pyrazolo[3, 4-g]quinoline). A D₃ receptor partial agonist is also able to partially inhibit the response produced by dopamine or its full agonists. *In vivo,* a DRD3 partial agonist produces dopaminomimetic responses, especially when dopamine is depleted such as in 6-hydroxydopamine-lesioned rats or in 1-methyl-4-phenyl-1,2,3,6-tetrahydropyridine (MPTP)-treated monkeys. Additionally, *in vivo* a DRD3 partial agonist may act as an antagonist, especially when DRD3 is subject to a sustained stimulation by dopamine. Examples of DRD3 partial agonists include BP 897 (1-(4-(2-Naphthoylamino)butyl)-4-(2-methoxyphenyl)-1A-piperazine, Pilla et al., 1999) and aripiprazole (7-[4-[4-(2, 3 dichlorophenyl)-1-piperazinyl]butoxy]-3, 4-dihydro-2(1*H*)-quinoline (Lawler et al., 1999).

A "D₃ receptor antagonist" denotes a molecule that forms a complex with DRD3 and that is capable of inhibiting an active response triggered by dopamine or its agonists from a cell expressing DRD3. Examples of D₃ receptor antagonists include nafadotride (N[(n-butyl-2-pyrrolidinyl)methyl]-1-methoxy-4-cyano naphtalene-2-carboxamide, Sautel et al. 1995), ST 198 ((E)-N-(4-[1,2,3,4-tetrahydroisoquinolin-2-yl]-butyl)-3-phenylacrylamide), SB-277701A (*trans-N-*[4-[2-(6-cyano-1,2,3,4-tetrahydroisoquinolin-2-yl)ethyl]cyclohexyl]-4-quinolinecarboxamide, Reavill et al., 2000), S33084 ((3*aR*,9*bS*)-*N*-[4-(8-cyano-1,3a,4,9b-tetrahydro-3H-benzopyrano[3,4-c]pyrrole-2-yl)butyl]'-phenyl) benzamides, Millan et al., 2000).

Advantageously the DRD3 partial agonist or antagonist to be administered may have been characterized for anti-DRD3 activity *in vivo.* This may be carried out according to a screening method as described in the international patent application PCT/US 02/31290. Briefly this screening method relies on the acute or continuous administration of a mammal with a low dose of NMDA antagonist to induce a psychotic behavior which is mediated by DRD3. The capacity of a ligand administred to the mammal to alleviate the psychotic behavior is then assessed and is indicative of a ligand with anti-DRD3 activity *in vivo.*

A dopamine D3 receptor partial agonist or antagonist may be preferably selected from the group consisting of BP 897, aripiprazole, nafadotride, ST 198, SB-277701A and S33084. Still preferably said dopamine ligand is the partial agonist BP 897.

In the above therapeutic methods or applications, the dopamine D3 receptor ligand, and in particular D3 receptor partial agonist or antagonist, may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

Such pharmaceutical compositions containing a ligand of the dopamine D3 receptor alone or in combination with another D3 ligand or another active ingredient may thus be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono-or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

A D3 ligand, such as a partial agonist or antagonist of the D3 receptor may be administered in any of the foregoing compositions and according to dosage regimens established in the art whenever specific blockade of the human D3 receptor is required.

The daily dosage of the products may be varied over a wide range from 0.01 to 1,000 mg per adult human per day. For oral administration, the compositions are preferably provided in the form of tablets containing 0.01, 0.05, 0.1, 0.5, 1.0, 2.5, 5.0, 10.0, 15.0, 25.0, 50.0, 100, 250 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. An effective amount of the drug is ordinarily supplied at a dosage level of from about 0.0002 mg/kg to about 20 mg/kg of body weight per day. Preferably, the range is from about 0.001 to 10 mg/kg of body weight per day, and especially from about 0.001 mg/kg to 7 mg/kg of body weight per day. The compounds may be administered on a regimen of 1 to 4 times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The invention will be further illustrated by the following figures and examples.

### FIGURES

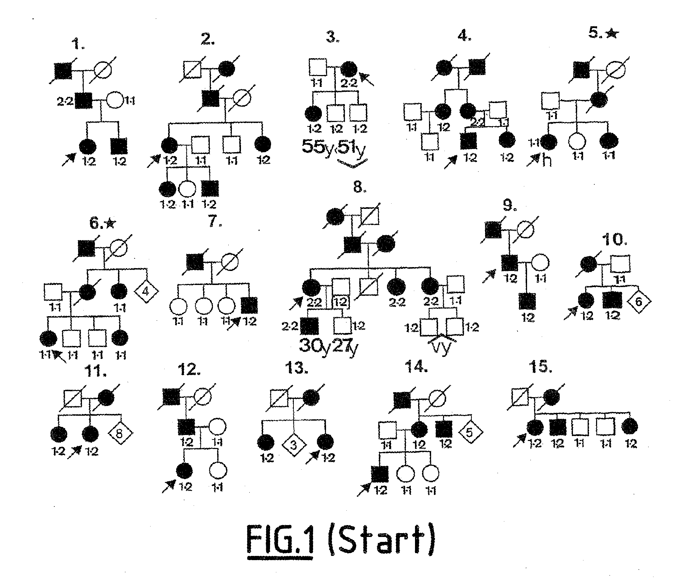

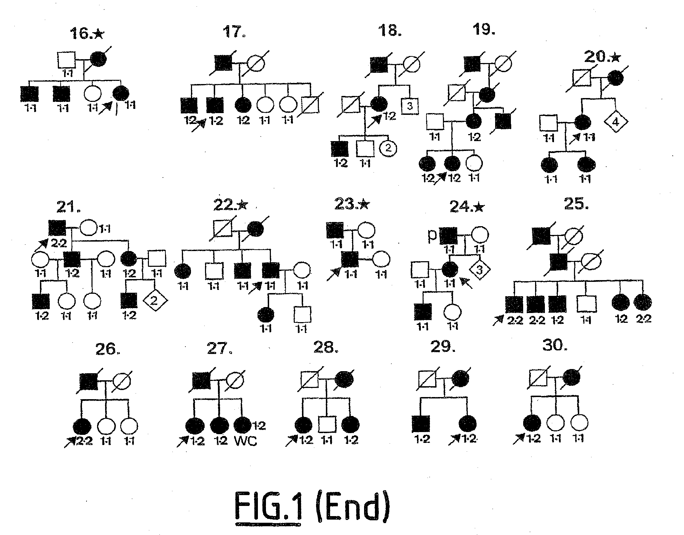

Figure 1 shows the cosegregation of allele 2 with ET in the 30 French ET families enrolled in the study. Arrows indicate index cases. The genotyped patients are either 1-1 homozygous, 1-2 heterozygous or 2-2 homozygous (in family 8 the genotype of the husband of the index patient is deduced). Allele 2 cosegregates with ET in all families where it is present, but not (penetrance effect with age) in family 3 (in the second generation the 1-2 patient is 55 years old and the two brothers are < 51 years) and in family 8 (in the fourth generation the 2-2 patient is 30 years old, the 1-2 brother is 27 years old, and the two 1-2 cousins are very young). In family 27 the third sister at the second generation is a special case of "writer's cramp" (wc). The seven families indicated with stars (families 5, 6, 16, 20, 22, 23, and 24) don't segregate for the genetic marker, because all genotypes members are homozygous 1-1. Among them, in family 5 the index case had tremor symptoms at the head-only (h), in family 16 the index case had tremor symptoms predominantly at the lip (Ip, and the father of the index patient in family 24 is a probable case of Parkinson (p).
Figure 2 illustrates the differences in mean of age of onset and in distribution between heterozygous 1-2 and homozygous 2-2 ET patients.
Figure 3 illustrates that the severity of tremor (forearm tremor score) is greater in homozygous 2-2 ET patients than in heterozygous 1-2.
Figure 4 illustrates the higher dopamine affinity for the ⁹Gly variant (allele 2) of the D₃ receptor than the ⁹Ser variant (allele 1).
Figure 5 illustrates that the response induced by dopamine, which is measured by inhibition of cyclic AMP accumulation, is twice as high at the ⁹Gly variant (allele 2) as at the ⁹Ser variant.

### EXAMPLES

Throughout the specification, allele 1 denotes the allele that harbours a A in position 31 and encodes a DRD3 protein with Ser in position 9. Allele 2 denotes the allele that harbours a G in position 31 and encodes a DRD3 protein with Gly in position 9. Accordingly genotype 1-2 is intended for heterozygous patients A-G (Ser-Gly) and genotype 2-2 denotes homozygous patients G-G (Gly-Gly).

First, a PCR assay was used for detecting the coding sequences for the functional Ser9Gly polymorphism in the N-terminal extracellular domain of DRD3 in 25 consecutive index ET familial unrelated cases, compared to controls. Thirteen cases, (52 %) were heterozygous 1-2 and six cases (24 %) were homozygous 2-2, those proportions being significantly different from those of controls. Allele 2 frequency in patients was significantly greater (p=0.001) than in controls, and in the study, the relative risk to be ET in subjects bearing at least one allele 2 is =2.92.

Secondly, a segregation study was carried out in thirty unrelated French ET families, each of them having a definite diagnosed clinical ET patient as index case. Allele 2 is associated with ET in 23 families out of 30. In all families where allele 2 is present, this allele cosegregates perfectly in patients (taking account of the gene penetrance with age). Homozygous 2-2 (Gly-Gly) patients presented with a more severe or a more precocious form of the disease, suggesting a gene dosage effect. The transmission/disequilibrium test (TDT) were used in families with at least one heterozygous 1-2 (Ser-Gly) parent, and gives a chi-sq. value = 5.444. Sib-TDT test (S-TDT) was also used in families where genotypes of parents were unavailable, and gives mean = 27.483, variance = 3.207 and z' = 5.016. The two combined scores = 5.344 (p=10⁻⁵).

Accordingly, these results indicate that the presence of allele 2 is the hallmark for an increased risk of developing ET. Furthermore, since the S9G mutation has been associated with a functional effect that suggests a gain-of-function mutation (Lundström and Turpin, 1996), this may explain why anti-parkinsonian agents that stimulate DRD3 (Sokoloff et al., 1990; Bézard et al., 2003) lack efficiency in the treatment of ET.

### EXAMPLE 1 : Association study

### Subjects and methods

### Patients and controls

Patients were examined by neurologists with a specific expertise of ET during 1999-2003. The protocol included neurological examination, tremor type and its topography, search for associated signs or symptoms, drugs in used and paraclinical investigations to define the diagnosis. 25 successive index cases (9 men and 16 women), from 25 unrelated families with familial ET underwent DNA analysis. All subjects fulfilled the diagnostic criteria of classic hereditary ET as defined by the Consensus Statement of the Movement Disorder Society (Deuschl et al., 1998) and only patients with probable and definite familial ET were included in the study (Watner et al., 2002). The inventors in their study adopted the classification of clinical subtypes (differences in anatomic distribution of tremor, in age of onset and in rate of progression) of ET (Louis et al., 2000).

Fifty healthy subjects served as pair controls, patients and controls being matched for sex and age (all cases and controls originated from France).

### Molecular analysis

After informed consent, genomic DNA was prepared for leucocytes harvested from whole blood of subjects by using standard methods involving proteinase K digestion and several phenol-chloroform extractions.

PCR amplification was carried out using a PCT-200 thermocycler, with the following primers flanking exon 1 of the D3 receptor gene : 2350A : 5'-GCTCTATCTCAACTCTCACA-3' (forward, SEQ ID No:3) and 2351B : 5'-AAGTCTACTCACCTCCAGGTA-3' (reverse, SEQ ID No:4) designated to amplify a specific 304 bp segment. PCR amplification was performed in a total volume of 50 µl containing 100 ng of genomic DNA, 1 U taq polymerase, 2 µl 10xPCR buffer, 10 pmol of each primer and 200 mM of each dNTP. Following denaturation of 95°C for 5 minutes, thirty cycles were performed with the profile of 92°C for 30 seconds, 56°C for 40 seconds, 72°C for 40 seconds, and this profile was followed by an extension at 72°C for 5 minutes.

After *Ms*cl restriction, 3 genotypes can be observed among individuals on 1 % agarose minigel colored by ethidium bromide : genotype 1-1, Ser-Ser (304 bp band), genotype 1-2, Ser-Gly (304 bp band together with 206 bp band) and genotype 2-2, Gly-Gly (206 bp band).

### Results

The proportions of the different genotypes in the control population are shown in Table 1 below :

**Table 1 :**

| percentages of genotypes in controls and patients | | | | | |
|---|---|---|---|---|---|
| | | Genotypes | | | |
| | # | 1-1 | 1-2 | 2-2 | X²(HWE) |
| Controls | 50 | 30 (60 %) | 14 (28 %) | 6 (12 %) | 2.89, NS |
| Patients | 25 | 6 (24 %) | 13 (52 %) | 6 (24 %) | 5.77, S |
| # : number of individual tested | | | | | |
| HWE : Hardy-Weinberg equilibrium | | | | | |
| S : significant | | | | | |
| NS : non-significant | | | | | |

These proportions are in Hardy-Weinberg equilibrium (HWE) relative to allele frequencies (X² = 2.89 < 5.99, d.f. = 2). These proportions in patients are: 6 (24 %), 1-1, 13 (52 %) 1-2 and 6 (24 %) 2-2, in significant (X² = 5.77 > 3.84, d.f. = 1, p < 0.02) HW disequilibrium, because of great heterozygous 1-2 and homozygous 2-2 excess.

Table 2 summarizes the genotype of the 25 unrelated consecutive index patients studied and their characterisations relative to sex, actual age, age on onset and anatomical localization of tremor.

**Table 2 :**

| Characterizations of the 25 consecutive ET patients studied | | | | | |
|---|---|---|---|---|---|
| Patient | Sex | Actual age (years) | Age of onset (years) | Anatomical localization of tremor | Genotypes |
| 1 | F | 74 | 68 | A, H | 1-2 |
| 2 | F | 66 | 60 | A, H | 1-2 |
| 3 | F | 82 | 20 (p) | A (s) | 2-2 |
| 4 | M | 51 | 35 | A | 1-2 |
| 5 | F | 69 | 64 | H | 1-1 |
| 6 | F | 73 | 12 (p) | A, H | 1-1 |
| 7 | M | 74 | 68 | A | 1-2 |
| 8 | F | 58 | 35 | A,H | 2-2 |
| 9 | M | 84 | 65 | A | 1-2 |
| 10 | F | 83 | 63 | A | 1-2 |
| 11 | F | 69 | 55 | A (H) | 1-2 |
| 12 | F | 46 | 18 (p) | A, V | 1-2 |
| 13 | F | 74 | 55 | A, (H) | 1-2 |
| 14 | M | 38 | 8 (p) | A | 1-2 |
| 15 | F | 66 | 56 | A | 1-2 |
| 16 | F | 69 | 64 | I | 1-1 |
| 17 | M | 69 | 58 | A | 1-2 |
| 18 | F | 66 | 55 | A | 1-2 |
| 19 | F | 17 | 12 (p) | A | 2-2 |
| 20 | F | 74 | 66 | A, H | 1-1 |
| 21 | M | 82 | 12 (p) | A, (s) | 2-2 |
| 22 | M | 65 | 58 | A | 1-1 |
| 23 | M | 51 | 40 | A | 1-1 |
| 24 | M | 30 | 7 (p) | A | 2-2 |
| 25 | F | 71 | 52 | A, (H), L | 2-2 |
| F : female ; M : male ; (p) : precocious ; A : arms ; H : head and voice ; V : voice only ; (H) : head only ; I : lips ; L : legs ; (s) : severe. | | | | | |

Most of the 13 heterozygous 1-2 (patients 1, 2, 7, 9, 10, 11, 13, 15, 17 and 18) present the "classical" form of the disease (age of onset ≥ 55 years, arm tremor progressing relatively quickly to arm + head tremor).

Allele 2 frequency is significantly greater (X² = 3.39 > 1.96, p=0.001) in patients (= 0.50) compared to controls (=0.26). In this study the relative risk to develop ET in subjects bearing at least one allele 2 is (19/39) (6/36) = 2.92.

### EXAMPLE 2 : Segregation study

### Patients and families

30 patients (index cases) belonging to unrelated French families (with positive family history) were included in the present study. Informed consents were obtained from each of them, before all clinical and genetic tests. All patients fulfilled the Tremor Investigation group Criteria (Deuschl et al., 1998) for the diagnosis of definite ET. The screening procedure for signs of ET consisted of the drawing of an Archimede spiral with the dominant hand within preprinted lines (Louis et al., 1996), and a clinical examination. A total of 51 ET patients (other than the index cases) and 58 non-ET subjects of these 30 families were included in the genetic study (Figure 1).

### Molecular analysis

PCR amplification was carried out as described above (Example 1). The PCR products were then restricted with *Ms*cl for 4 hours at 37°C, and digestion products were analyzed following electrophoresis in 3 % Metaphor agarose gel stained with ethidium bromide under UV light.

Restriction results in a 304 bp product (allele 1), or a 206 bp (allele 2) and a 98 bp product, as well as constant bands of 111 bp and 47 bp.

### Cosegregation between allele 2 and ET

Allele 2 segregates in 23 families out of 30 (≈ ¾). Cosegregation between allele 2 and ET was observed (Figure 1) in these 23 families. Some effects of penetrance with age is manifest in family 3 (in the second generation the heterozygous ET patient is 55 years old and the two brothers are < 31 years) and in family 8 (in the fourth generation the homozygous ET patient is 30 years old, the heterozygous brother is 27 years old and the two heterozygous patients are very young).

### Linkage analysis

Family-based association between allele 2 and ET was evaluated by the TDT transmission/disequilibrium test (Spielman et al., 1993) in families with at least one heterozygous parent, which lead to a Chi-sq. value = 8.333 (p=0.0039). A Sib-TDT (S-TDT) analysis was also performed in sibships without prenatal datas (Spielman and Ewens, 1998). Values obtained are: Mean (A) = 14.017, variance (V) = 2.380, and z' = 4.203 (p < 0.0001). The two values combined are : A = 20.017, V = 5.380, and the z' combined score = 4.951 (p < 10⁻⁵). Table 3 below shows TDT and S-TDT values when the husband of the index case in family 8 is non-inferred :

**Table 3 :**

| TDT | | | | |
|---|---|---|---|---|
| Allele | b | C | Chi-Sq. | |
| 1 | 1 | 10 | 7.364 | |
| 2 | 10 | 1 | 7.364 | p = 0.0067 |

| S-TDT | | | | |
|---|---|---|---|---|
| Y | Mean (A) | Variance (V) | z' | |
| 13 | 19.983 | 2.380 | 4.203 | |
| 21 | 14.017 | 2.380 | 4.203 | p < 0.0001 |

| Combined scores | | | | |
|---|---|---|---|---|
| W | Mean (A) | Variance (V) | z' | |
| 14 | 25.483 | 5.130 | 4.849 | |
| 31 | 19.517 | 5.130 | 4.849 | p < 0.0001 |

Linkage of allele 2 and ET was also demonstrated by parametric linkage analysis in these families, assuming a penetrance of 0.90, which is similar to published data on familial ET and compatible with the observed transmission in our families. Analyses assuming a rate of phenocopies ranging from 0.01 to 0.10 and a frequency of the mutated allele ranging from 0.01 to 0.50 yielded LOD scores ranging from 5.38 to 7.99 at θ = 0.

### Age at onset in allele-2 heterozygous and homozygous

Table 4 below summarizes age at onset of the disease in heterozygous 1-2 and homozygous 2-2 patients (index cases) in the 23 ET families where allele 2 segregates.

**Table 4 :**

| Ages at onset (in years) | | |
|---|---|---|
| Families | 1-2 genotype | 2-2 genotype |
| 1 | 74 | |
| 2 | 66 | |
| 3 | | 20 |
| 4 | 35 | |
| 7 | 68 | |
| 8 | | 10 |
| 9 | 65 | |
| 10 | 63 | |
| 11 | 55 | |
| 12 | 18 | |
| 13 | 55 | |
| 14 | 8 | |
| 15 | 56 | |
| 17 | 58 | |
| 18 | 55 | |
| 19 | | 13 |
| 21 | | 16 |
| 25 | | 7 |
| 26 | | 52 |
| 27 | 22 | |
| 28 | 17 | |
| 29 | 61 | |
| 30 | 60 | |

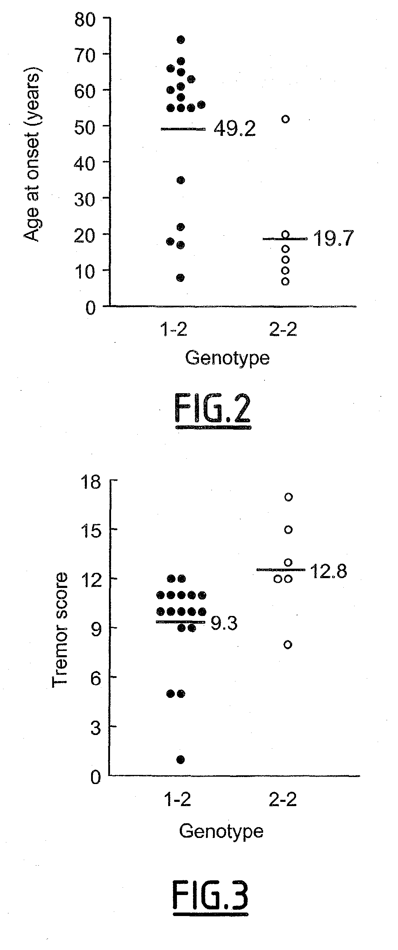

In the 6 homozygous patients, the mean age at onset = 19.7 years, and in the seventeen heterozygous patients the mean age at onset = 49.2 years (Figure 3). The two means are significantly different (ε = 3.52, p < 0.001), homozygosity being clearly associated with early onset of the disease.

### Severity of symptoms in allele-2 heterozygous and homozygous

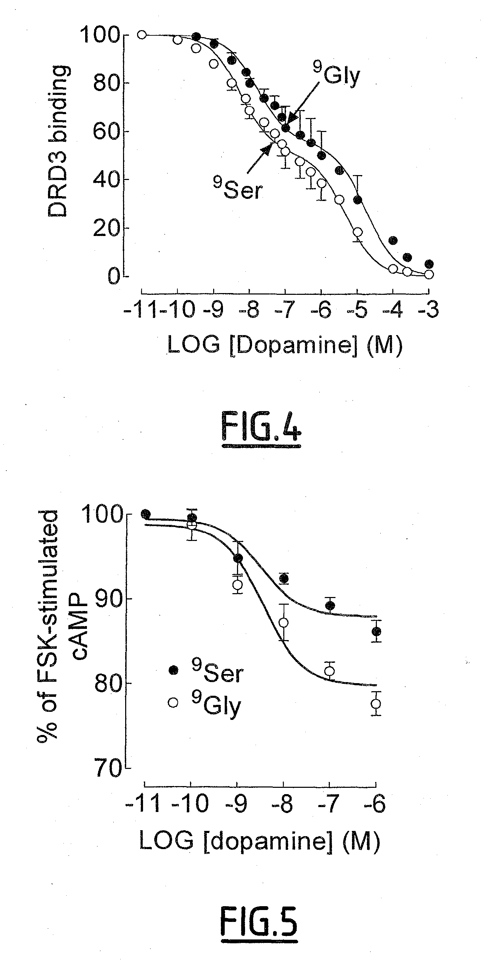

Probands homozygotes for allele 2 have more severe symptoms than heterozygotes. For instance, Table 5 shows that forearm tremor scores were higher (Figure 4). The mean tremor scores are 9.29 ± 2.9 (mean ± S.D.) and 12.83 ± 3.06 in 17 heterozygous and 6 homozygous patients, respectively. The difference is significant (two-tailed P = 0.0157 by the Mann-Whitney *U* test).

**Table 5:**

| forearm tremor scores | | |
|---|---|---|
| Families | 1-2 genotype | 2-2 genotype |
| 1 | 11 | |
| 2 | 10 | |
| 3 | | 13 |
| 4 | 10 | |
| 7 | 12 | |
| 8 | | 8 |
| 9 | 11 | |
| 10 | 11 | |
| 11 | 11 | |
| 12 | 5 | |
| 13 | 10 | |
| 14 | 5 | |
| 15 | 11 | |
| 17 | 9 | |
| 18 | 12 | |
| 19 | | 12 |
| 21 | | 12 |
| 25 | | 15 |
| 26 | | 17 |
| 27 | 1 | |
| 28 | 10 | |
| 29 | 9 | |
| 30 | 10 | |

Although several localizations were proposed, most families studied until now segregates for an ET gene located in 3q13 (Gulcher et al., 1997). As reported herein, the dopamine D3 receptor (DRD3) gene was tested as a potential candidate gene for ET. More specifically, a PCR assay was carried out to amplify the part of the DRD3 gene coding for the two *Ms*cl alleles (alleles 1 and 2), corresponding to the Ser-9-Gly polymorphism in the N-terminal extracellular domain of the receptor protein D3.

The inventors found a remarkable association between allele 2 and the ET gene in 25 consecutive index ET patients of unrelated ET families where the disease is transmitted in a dominant fashion compared to controls : 19 of them (76 %) bears at least one copy of the rarest allele 2. If most of the heterozygous 1-2 patients presented the common ET phenotype, a gene dosage effect is manifest because homozygous patients 2-2 have some severe and/or precocious form of the disease. Overall allele 2 confers relative risk of about 3 in patients bearing at least one of this allele.

### EXAMPLE 3 : Functional study

The *in vivo* functional significance of the polymorphic S9G substitution in the N-terminal extracellular domain of the DRD3 was investigated in recombinant cells expressing each of the two DRD3 variants.

### Plasmid contructs

A plasmid pRc/CMV bearing a cDNA encoding the ⁹Ser variant (Pilon et al., 1994) was used to generate cDNA encoding the ⁹Gly variant by site-directed mutagenesis using the QuickChange® multi Site-directed Mutagenesis Kit (Stratagene).

### Cell culture and transfection

HEK293 cells were cultured in a cell culture medium made with a 1:1 mixture of Dulbecco's modified Eagle's medium (DMEM) and F-12 (Life Technologies), supplemented with 10 % fetal calf serum and 100 µg/ml penicillin/streptomycin in humidified atmosphere of 5 % CO₂, 95 % air. Cells were seeded in 10-cm dishes at 50-80 % confluency and cDNA encoding the ⁹Ser or the ⁹Gly variant was transfected (10 µg of DNA for 10⁶ cells) using Superfect® (Qiagen). Transfectants were selected with G418 (Invitrogen) at the concentration of 800 µg/ml. Two representative clonal cell lines were selected and used for binding and functional assays.

### Binding assay

Cells were detached from culture dishes in the presence of 0.2% trypsine, harvested by centrifugation and suspended in DMEM-F12 supplemented with ascorbic acid (50 µg/ml), and incubated for 30 min at 30°C with [¹²⁵I]iodosulpride (Amersham) at 0.1 nM and dopamine in increasing concentrations. Non specific binding was measured in the presence of enomapride (1 µM). Binding was stopped by filtration through GF/C filters coated with polyethylene imine (0.3 %) and radioactivity counted on the filter by gamma scintigraphy.

### Cyclic AMP accumulation assay.

HEK293 cells expressing the ⁹Ser or the ⁹Gly variant have been used. These cells were transiently transfected with cDNA encoding the adenylyl cyclase of type V (Salim et al., 2003). Cells were preincubated with 10 µM 3-isobutyl-1-methylxanthine in DMEM-F12 for 25 min and incubated with dopamine in increasing concentrations for 10 min in the presence of 0.5 µM forskolin. The reaction was stopped by addition of 50 µl of ice-cold 0.1 M HCl. The cells were mildly sonicated and cAMP accumulation was assayed with the Rianen [¹²⁵I]cAMP radioimmunoassay kit (DuPont/NEN).

### Dopamine affinity at the ⁹Ser and ⁹Gly variants.

Dopamine inhibited surface DRD3 binding in a biphasic manner (Figure 4), a feature common to G protein-coupled receptors (GPCR); the two sites corresponded to the high-affinity state, coupled to the G protein that transduces the signal to the effector, and to the low-affinity, uncoupled state. Each of the two sites was similarly affected by the mutation, which increased dopamine affinity by 4-5 times (P < 0.01), but did not change the proportion of each affinity site (45 ± 2% and 49 ± 2% for the 9Ser and 9Gly variants, respectively). These results show that dopamine had a higher affinity for the 9Gly variant, in agreement with a previous study (Lundstrom and Turpin, 1996), and indicate that the difference occurred at each of the two affinity sites, suggesting that the mutation did not affect the equilibrium between the G protein-coupled and uncoupled forms of the receptor, but rather the overall conformation of the receptor.

### Gain-of-function of the ⁹Gly variant

Stimulation of DRD3 by dopamine inhibits in a concentration-dependent manner forskolin-induced accumulation of cyclic AMP (Griffon et al., 1997). The maximal response of dopamine was twice as high at the ⁹Gly variant as at the ⁹Ser variant (Figure 5). These results show the gain-of-function produced by the S9G mutation.

### REFERENCES

- Barbas CF, Bain JD, Hoekstra DM, Lerner RA. (1992), Semisynthetic combinatorial antibody libraries: a chemical solution to the diversity problem. PNAS USA, 89, 4457-4461
- Black et al. (2002) A possible substrate for dopamine-related changes in mood and behavior: prefrontal and limbic effects of a D3-preferring dopamine agonist. Proc. Natl. Acad. Sci. USA. 99:17113-17118.
- Brin et al. (1998) Epidemiology and genetics of essential tremor. Mov Disor 13 (suppl 3):55-63
- Chomczynski, P. , Sacchi, N.(1987) Anal. Biochem., 162:156-159.
- Colas et al., (1996) Genetic selection of peptide aptamers that recognize and inhibit cyclin-dependent kinase 2. Nature. 380(6574):548-50
- Cooper et al. (1991) Diagnosis of genetic disease using recombinant DNA, 3rd edition, Hum. Genet, 87:519-560
- Deutschl et al. (1998) Consensus statement of the Movement Disorder Society on tremor. Mov Disord 13(suppl. 3):2-23
- Dunnen et Antonarakis (2000) Mutation nomenclature extensions and suggestions to describe complex mutations: a discussion. Hum Mutation. 15 :7-12; Erratum in: Hum Mutat 2002 ;20(5):403
- Farrer et al. (1999) A chromosome 4p haplotype segregating with Parkinson's disease and postural tremor. Hum Mol Genet 8:81-85
- Grompe M. The rapid detection of unknown mutations in nucleic acids (1993) Nat. Genet. 5(2):111-7
   Giros et al. (1990) [Gene cloning of human dopaminergic D3 receptor and identification of its chromosome] C R Acad Sci III. 311 (13):501-8
- Griffon et al. (1997). Two intracellular pathways for the dopamine D₃ receptor : opposite and synergistic interactions with cyclic AMP. J. Neurochem. 68:1-9.
- Gulcher et al. (1997) Mapping of a familial with essential tremor gene, FET1, to chromosome 3q13. Nat Genet. 17:84-87
   Ichiguro et al. (2000) Mutation and association analysis of the 5' region of the dopamine D3 receptor gene in schizophrenia patients: identification of the Ala38Thr polymorphism and suggested association between DRD3 haplotypes and schizophrenia. Mol. Psychiatry, 5:433-438
- Harlow E. et al., Antibodies: A Laboratory Manual, Cold Spring Harbor Laboratory, New York, (1988)
- Higgins et al. (1997) A gene (ETM) for essential tremor maps to chromosome 2p22.p25. Mov Disord 12:859-869
- Higgins et al. (1998) Evidence that a gene for ET maps to chromosome 2p in 4 families. Mov Disord 13:972-977
- Jayasena S.D. (1999) Aptamers: an emerging class of molecules that rival antibodies in diagnostics. Clin Chem. 45(9):1628-50
- Kohler and Milstein (1975) Continuous cultures of fused cells secreting antibody of predefined specificity. Nature ;256, 495-7
- Kuklin et al. Detection of single-nucleotide polymorphisms with the WAVE DNA fragment analysis system Genet. Test (1997-98), 1 (3):201-6
- Lannfelt et al. (1992) Amino acid substitution in the dopamine D3 receptor as a useful polymorphism for investigating psychiatric disorders. Psychiatric Genetics 2:249-256
- Lawler et al. (1999) Interactions of the novel antipsychotic aripripazole (OPC-14597) with dopamine and serotonin receptor subtypes. Neuropsychopharmacology 20:612-627
- Little et al. (1996) HLA-C typing of eleven Papua New Guineans: identification of an HLA-Cw4/Cw2 hybrid allele. Tissue Antigens. 48(2):113-7
- Louis et al. (1996) How familial is familial tremor? The genetic epidemiology of essential tremor. Neurology 46:1200-1205
- Louis et al. (1998) How common is the most common adult movement disorder? Estimates of the prevalence of essential tremor throughout the world. Mov Disord 13: 5-10
- Louis et al. (2000) Clinical subtypes of essential tremor. Arch Neurol 57:1194-1198
- Lundström and Turpin (1996) Proposed schizophrenia-related gene polymorphism : expression of the Ser9Gly mutant human dopamine D3 receptor with the Semliki virus system, Bioch. Biophys. Res. Comm. 225 :1068-1072
- Millan et al. (2000) S33084, a novel potent, selective, and competitive antagonist at dopamine D₃-receptors: receptorial, electrophysiological and neurochemical profile compared with GR218,231 and L741,626. The Journal of Pharmacology and Experimental Therapeutics 293, 1048-1062
- Nickerson et al., (1990) Automated DNA diagnostics using an ELISA-based oligonucleotide ligation assay. PNAS 87(22):8923-7
- Pilla, M., Perachon, S., Sautel, F., Garrido, F., Mann, A., Wermuth, C.G., Schwartz, J.-C., Everitt, B.J., Sokoloff, P., (1999) Selective inhibition of cocaine-seeking behaviour by a partial dopamine D₃ receptor agonist. Nature 400, 371-375
   Pilon et al. (1994) Functional coupling of the human dopamine D₃ receptor in a transfected NG 108-15 neuroblastoma-glioma hybrid cell line. Eur. J. Pharmacol. [Mol. Pharmacol. Sect.] 268:129-139.
- Reavill et al. (2000) Pharmacological actions of a novel high-affinity, and selective human dopamine D₃ receptor antagonist, SB-277011-A. The Journal of Pharmacology and Experimental Therapeutics 294, 1154-1165
- Salim et al. (2003) Identification of RGS2 and type V adenylyl cyclase interaction sites. J. Biol. Chem., 278:16842-15849.
- Sambrook, Fritsch & Maniatis, Molecular Cloning: A Laboratory Manual, Second Edition (1989) Cold Spring Harbor Laboratory Press, Cold Spring Harbor, New York
- Sautel et al. (1995) Nafadotride, a potent preferential dopamine D₃ receptor antagonist, activates locomotion in rodents. The Journal of Pharmacology and Experimental Therapeutics 275, 1239-1246
- Spielman et al. (1993) Transmission test for linkage disequilibrium : the insulin gene region and insulin-dependent diabetes mellitus Am J Hum Genet 52:506-516
- Spielman and Ewens (1998) A sibship test for linkage in the presence of association : the sib transmission/disequilibrium test Am. J. Hum. Genet. 62:450-458
- Tuerk C. and Gold L. (1990) Systematic evolution of ligands by exponential enrichment: RNA ligands to bacteriophage T4 DNA polymerase. Science. 3;249(4968):505-10
- Waterhouse P, Griffiths AD, Johnson KS, Winter G. (1993) Combinatorial infection and in vivo recombination: a strategy for making large phage antibody repertoires. Nucleic Acids Research, 21, 2265-2266
- Watner et al., (2002) Survey of essential tremor patients on their knowledge about the genetics of the disease. Mov Disord 17:378-381

## Claims

1. An *in vitro* method for diagnosing essential tremor, or a risk of developing essential tremor, which comprises detecting a mutation in the dopamine D3 receptor (DRD3) gene, said mutation being indicative of essential tremor or of an increased risk of developing essential tremor.

2. The method according to claim 1, wherein said mutation is selected from the group consisting of a missense mutation, a non sense mutation, a deletion, an insertion, or a combination thereof.

3. The method according to claim 1 or 2, wherein said mutation is in the regulatory region, in the coding region, or in the introns of DRD3 gene.

4. The method according to claim 3, wherein said mutation is in the coding region of DRD3 gene.

5. The method according to claim 4, wherein said mutation is a 31A>G substitution in the coding region of DRD3 gene.

6. The method according to claim 4 or 5, wherein said mutation is a 31A>G substitution in the DRD3 coding sequence as shown in SEQ ID No.1.

7. The method according to claim 4, wherein said mutation results in a mutation of DRD3 protein.

8. The method according to claim 7, wherein said mutation is a S9G substitution in DRD3 protein.

9. The method according to claim 7 or 8, wherein said mutation is a S9G substitution in the DRD3 amino acid sequence as shown in SEQ ID No.2.

10. An in vitro method for diagnosing essential tremor, or a risk of developing essential tremor, which comprises detecting an increase in DRD3 protein activity on a test subject compared with a control subject, wherein an increased DRD3 protein activity is indicative of essential tremor, or a risk of developing essential tremor.

11. The method according to claim 10, wherein the level of expression or activity of DRD3 protein is assessed in a test subject and compared to the level of expression or activity in a control subject, wherein an increased expression or basal activity of DRD3 protein in the test subject compared with the control subject is indicative of essential tremor, or a risk of developing essential tremor.

12. Use of an oligonucleotide that specifically hybridizes to or adjacent to a site of mutation of DRD3 gene for the in vitro diagnosis of essential tremor, or of a risk of developing essential tremor.

13. The use according to claim 12, wherein the mutation site is the nucleotide position 31 of DRD3 coding sequence as shown in SEQ ID No.1.

14. The use according to claim 12 or 13, wherein a pair of oligonucleotides consisting of SEQ ID No.3 and SEQ ID No.4 is used.

15. The use of a dopamine D3 receptor (DRD3) ligand having anti-D3 receptor activity for the manufacture of a medicament intended for the treatment of essential tremor.

16. The use according to claim 15, wherein said ligand is a partial agonist or an antagonist of DRD3.

17. The use according to claim 15, wherein said DRD3 partial agonist or antagonist is selected from the group consisting of BP 897 (1-(4-(2-Naphthoylamino)butyl)-4-(2-methoxyphenyl)-1A-piperazine), aripiprazole, 4-dihydro-2(1*H*)-quinoline), nafadotride, ST 198 ((E)-N-(4-[1,2,3,4-tetrahydroisoquinolin-2-yl]-butyl)-3-phenylacrylamide), SB-277701A *trans-N-*[4-[2-(6-cyano-1,2,3,4-tetrahydroisoquinolin-2-yl)ethyl]cyclohexyl]-4-quinolinecarboxamide) and S33084 ((3*aR*,9*bS*)-N-[4-(8-cyano-1,3*a*,4,9*b*-tetrahydro-3H-benzopyrano[3,4-*c*]pyrrole-2-yl)butyl]'-phenyl).
